# EUROPEAN PATENT SPECIFICATION

(11) **EP 1 574 324 B1**
(45) Date of publication and mention of the grant of the patent: **30.09.2009**
(21) Application number: 05251482.5
(22) Date of filing: 11.03.2005
(51) Int. Cl.: B32B 3/10, A61F 13/15, B29C 51/00

(54) **Three dimensional apertured film**
Dreidimensionaler perforierter Film
Film tridimensionel à ouvertures

(30) Priority: 12.03.2004 US 800092
(43) Date of publication of application: 14.09.2005
(73) Proprietor: McNeil-PPC, Inc., Skillman, New Jersey 08558 (US)
(72) Inventor: Kelly, William G. F., Middlesex, NJ 08846 (US)
(74) Representative: Mercer, Christopher Paul

(56) References cited:
- EP-A- 1 430 861
- WO-A-93/12749
- WO-A-99/62449
- US-A1- 2004 043 189

## Description

### Field of the Invention

The present invention relates generally to three-dimensional apertured film materials useful as components of personal care products such as sanitary napkins, diapers, incontinence products, tampons, surgical dressings, wound dressings, underpads, wiping cloths, and the like. More particularly, the present invention relates to three-dimensional apertured polymeric films with improved fluid-handling and masking properties when used as a component layer in a disposable absorbent article.

### Background of the Invention

The use of apertured films in personal care products is well known in the art. These films may be used as body-contacting facing layers, as fluid handling layers or as other components of personal care products. When such films are used in feminine sanitary protection articles as the body-contacting facing layer, it has been generally found that the higher the open area of the film the more effectively the film will transfer menstrual fluid to underlying layers (e.g. transfer layer, absorbent core) of the article. Unfortunately, it has also be found that the higher the open area of the film, the less effective the film is at stain "masking" the absorbed menstrual fluid stain once the menstrual fluid has been transferred to the underlying layers of the article. That is, the higher the open area of the film, the more visible the menstrual fluid stain will be after it is absorbed by the article.

US patent application number 10/404,367 (published as US 2004/0043189) and

European patent application number 02258797.6 (EP 1430861) disclose a perforated deformable web with three imaginary surfaces, a plurality of apertures and a plurality of members spanning each aperture thus dividing them into smaller apertures. The resultant plurality of recesses and capillaries extend from at least one recess which has a cross section greater than that of the cross section of the capillaries.

International patent application WO 93/12749 discloses a resilient plastic web comprising a plurality of apertures and a highly limited planar portion on its uppermost (fluid receiving) surface.

WO 99/62449 discloses a three dimensional elastomeric web comprising a plurality of apertures created by a series of interconnecting members. The webs disclosed in the above documents are all disclosed as being suitable for use in personal care products in which fluid must be moved and/or stored.

It is the object of the present invention to provide an apertured film having improved fluid-handling properties when used in disposable absorbent articles such as, for instance, feminine sanitary protection products. More particularly, it is an object of the present invention to provide an apertured film that effectively transfers fluid to an underlying absorbent structure while at the same time exhibits improved stain masking characteristics.

### Summary of the Invention

In view of the foregoing, the invention provides a three dimensional apertured film comprising: a first planar surface in a first imaginary plane; a second planar surface in a second imaginary plane located below said first imaginary plane; a first plurality of apertures; at least one member spanning each one of said first plurality of apertures to thereby define a plurality of smaller apertures, each of said plurality of smaller apertures in communication with a respective one of said first plurality of apertures, wherein said member spanning each one of said apertures has a top surface located in a third imaginary plane, said third imaginary plane being located below said first imaginary plane and said second imaginary plane, and a bottom surface located in a fourth imaginary plane located below the first, second and third imaginary planes.

### Brief Description of the Figures

Fig. 1a is a schematic view of a three-dimensional film according to one embodiment of the present invention;
Fig. 1b is a sectional view taken along line B as indicated in Fig. 1a;
Fig. 1c is an enlarged photomicrograph of the three-dimensional film schematically shown in Fig. 1a, showing a top surface thereof;
Fig. 1d is an enlarged photomicrograph of the three-dimensional film schematically shown in Fig. 1b, showing a bottom surface thereof;
Fig. 2 is a schematic illustration of one type of three dimensional topographical support member useful to make a film of the present invention;
Fig 3 is a schematic illustration of an apparatus for laser sculpting a workpiece to form a three dimensional topographical support member useful to make a film of the present invention.
Fig. 4 is a schematic illustration of a computer control system for the apparatus of Fig. 3;
Fig. 5 is a graphical representation of a file to laser sculpt a workpiece to produce a three dimensional topographical support member for producing an apertured film shown in Figs. 1a-1d;
Fig. 5a is a graphical representation of the file shown in Fig. 5 showing an enlarged portion thereof;
Fig. 6 is a photomicrograph of a workpiece after it was sculpted utilizing the file of Fig. 5;
Fig. 7 is a view of a support member used to make a film according to the invention in place on a film-forming apparatus;
Fig. 8 is a schematic view of an apparatus for producing an apertured film according to the present invention;
Fig. 9 is a schematic view of the circled portion of Fig. 8;
Fig. 10 is an average histogram representing stain intensity for an absorbent article having an apertured film according to the present invention as the cover layer thereof; and
Fig 11 is a graphical representation of a file to drill a workpiece using raster scan drilling to produce a three dimensional topographical support member for producing an apertured film.

### Detailed Description of the Invention

The present invention is directed to three-dimensional apertured films particularly useful in personal care products. These films may be used as body-contacting facing layers, as fluid handling layers, or as other components of personal care products. The films of the present invention have been found to exhibit improved fluid-handling properties when used in disposable absorbent articles such as, for instance, feminine sanitary protection products. In particular, the films of the present invention have been found to provide improved stain masking characteristics while at the same time permitting the efficient transfer of fluid through the film as compared to conventional films.

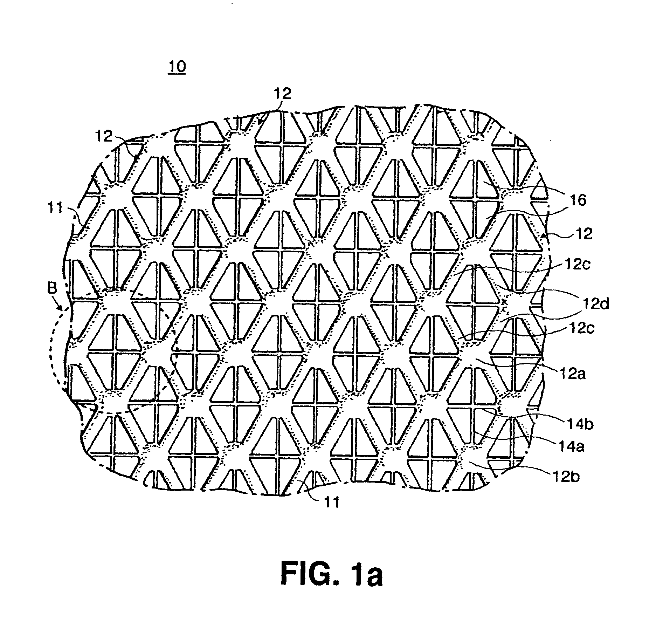

Reference is now made to Figs. 1a-1d which depict an apertured film 10 according to one embodiment of the present invention. The film 10 includes a plurality of repeating interconnected frames 12. In the embodiment shown in Figs. 1a-1e, each frame 12 includes opposed end regions 12a and 12b and opposed side walls 12c and 12d. Each of the end regions 12a and 12b being in spaced relationship to one another and each of the opposed side walls 12c and 12d being in spaced relationship to one another. In the specific embodiment shown in Figs. 1a-1d, each of the frames 12 are interconnected to an adjacent frame 12. More particularly, as shown, each frame 12 "shares" a common side wall 12c, 12d, with a directly adjacent frame 12. Likewise, each frame 12 shares a common end region 12a, 12b with a directly adjacent frame 12. The apertured film 10 further includes first and second cross members 14a and 14b. As shown, cross member 14b extends from a first side wall 12c to an opposed side wall 12d of the frame 12. Likewise, cross member 14a extends from an end region 12a to the opposed end region 12b. In the embodiment of the invention shown in Figs. 1a-1e, the cross members 14a and 14b intersect at the center of the frame is shown. In addition, in the embodiment of the invention shown in Figs. 1a-1e, the cross members 14a and 14b are arranged such that they are orthogonally arranged to one another.

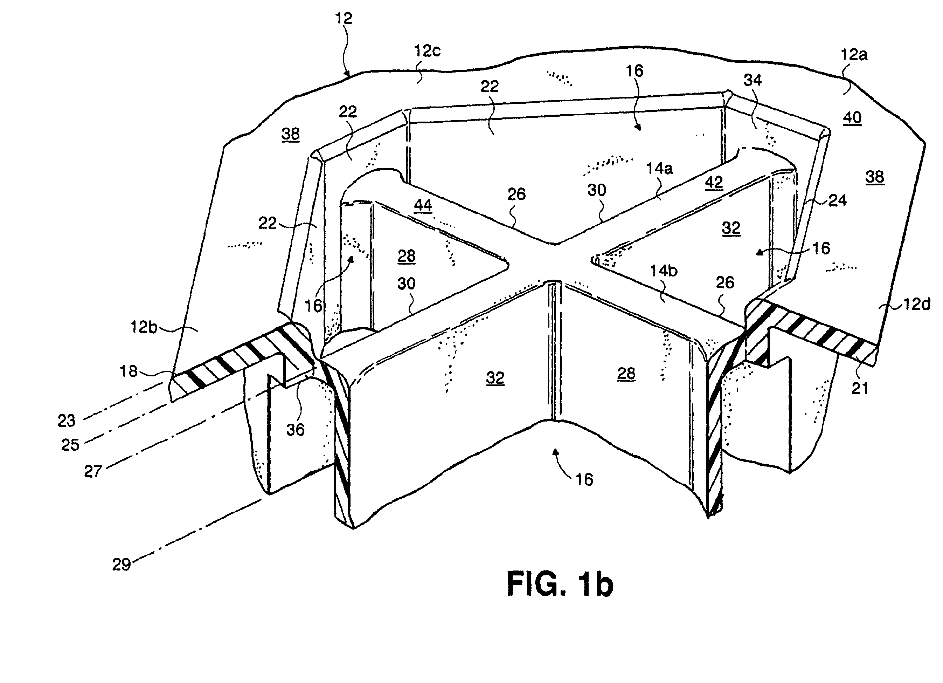

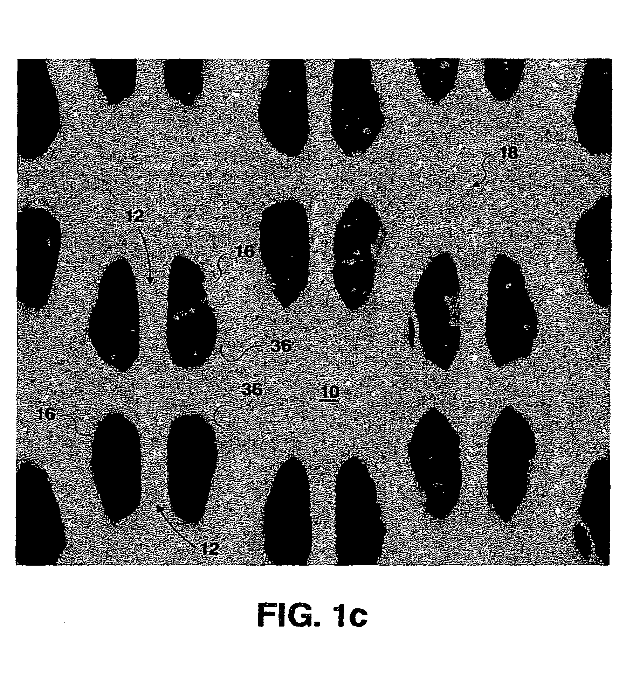

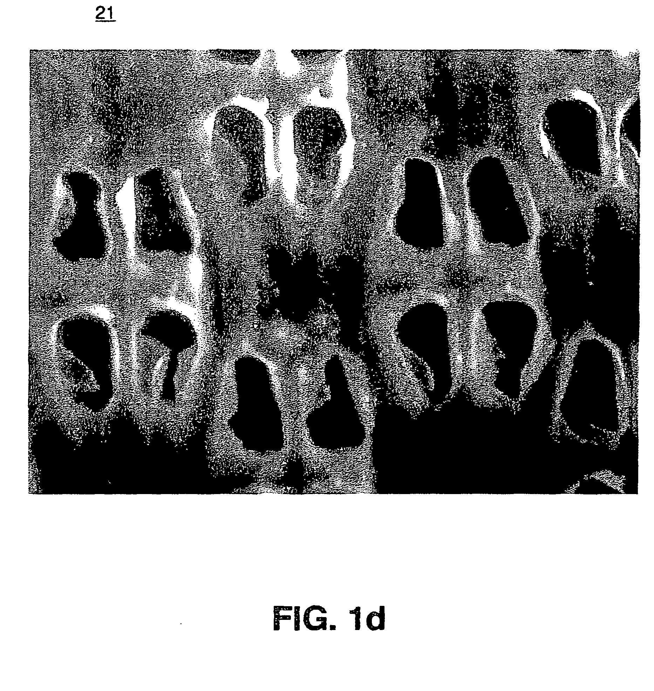

Although the embodiment of the invention shown in Figs. 1a-1d shows the apertured film as having two cross members 14a and 14b, it is possible that only a single cross member could be employed as long as the cross member extends substantially across an open area defined by the frame 12. Also, although the frame 12 has been shown as being generally hexagonal in shape, it is possible that other shapes be used for the frame 12. The cross members 14a and 14b preferably have a width in the range of about 203.2 nm (.008 mils) to about 609.6 nm (.024 mils). The film 10 may optionally include a plurality of bumps 11 or the like arranged on the surface of the film as best seen in Fig. 1 a.

The film 10 further includes a plurality of apertures 16. Each aperture 16 is bound by at least a portion of the frame 12 and at least a portion of one of the cross members 14a and 14b. Reference is now made to Fig. 1b which is an illustration of a cross-section of the film 10 shown in Fig. 1 taken along line A of Figure 1a. Each aperture is bound by at least a portion of each of the cross members 14a and 14b as well as by a portion of the frame 12. More particularly, as best seen in Fig. 1b, each of the apertures 16 is bound by a corresponding interior wall 22, 24 of a respective side wall 12c, 12d of the frame portion 12. Each aperture 16 is further bound by a corresponding interior wall 26 or 28 of cross member 14b and a corresponding interior wall 30, 32 of cross member 14a. Finally, each aperture 16 is bound by a respective interior wall 34, 36 of a corresponding end region 12a, 12b.

Again referring to Fig. 1b, film 10 generally includes a first generally planar top surface 18 in imaginary plane 23 and an opposed, generally planar, second bottom surface 21 in imaginary plane 25. The top surface 38 of the side walls 12c and 12d and the top surface 40 of the end regions 12a and 12b are coplanar with plane 23. However, the top surfaces 42 and 44 of cross members 14a and 14b are recessed relative to plane 23. More particularly, the top surfaces 42 and 44 of cross members 14a and 14b are located in a plane 27 located below both planes 23 and 25. Preferably the top surfaces 42 and 44 of the cross members 14a and 14b are recessed relative to the top surface 18 of the film, i.e. recessed relative to plane 23, to a depth in the range of about 127 µm (5.0 mils) to about 432 µm (17.0 mils). The top surfaces 42 and 44 of cross members 14a and 14b are preferably substantially parallel to the imaginary planes 23 and 25.

As best seen in Fig. 1c and 1d, interior walls 22, 24 of side walls 12c and 12d, interior walls 26, 28 of cross member 14a, interior walls 30, 32 of cross member 14b, and interior walls 34, 36 of end regions 12a, 12b cooperate to define the apertures 16 and each of these interior walls extend below plane 25 such that the bottom opening of each aperture 16 is located below the bottom planar surface 21 of the film, i.e., below imaginary plane 25. More specifically, interior walls 22, 24 of side walls 12c and12d, interior walls 26, 28 of cross member 14a, interior walls 30, 32 of cross member 14b, and interior walls 34, 36 of end regions 12a, 12b extend downwardly such that the bottom opening of each aperture is located in imaginary plane 29 which is located below imaginary planes 23, 25 and 27. It is noted that imaginary planes 23, 25, 27 and 29 are all substantially parallel to one another.

Since the top surfaces 42, 44 of the cross members 14a and 14b are recessed relative to the top surface 18 of the film 10, i.e., recessed relative to imaginary plane 23, a first relatively large aperture is effectively defined from the top surface 18 of the film 10 to the top surfaces 42, 44 of the cross members. The cross members 14a and 14b act to divide this larger aperture into four relatively smaller apertures which are in communication with the larger aperture from the top surfaces 42, 44 of the cross members 14a and 14b through the bottom opening of each aperture 16. Stated another way, within each frame member 12, a relatively large aperture is defined from plane 23 to plane 27 and a plurality of relatively smaller apertures, that are communication with the larger aperture, are defined from plane 27 to plane 29. In the embodiment shown in Figs. 1a-1d, each of the smaller apertures defined from plane 27 to plane 29 have an area that is less than one quarter of the total area of the larger aperture defined from plane 23 to 27. In an embodiment in which a single cross member was employed, each of the smaller apertures defined by the cross member would have an area less than one half the total area of the larger aperture. The reader is advised that for simplicity and clarity in the drawings, both the "smaller" and "larger" apertures discussed above are generally identified by reference numeral 16 herein.

The apertured films according to the present invention preferably have an open area in the range about 20% to about 30%. Open area may be determined by using image analysis to measure the relative percentages of apertured and unapertured, or land, areas. Essentially image analysis converts an optical image from a light microscope into an electronic signal suitable for processing. An electronic beam scans the image, line-by-line. As each line is scanned, an output signal changes according to illumination. White areas produce a relatively high voltage and black areas a relatively low voltage. An image of the apertured formed film is produced and, in that image, the holes are white, while the solid areas of thermoplastic material are at various levels of gray.

The more dense the solid area, the darker the gray area produced. Each line of the image that is measured is divided into sampling points or pixels. The following equipment can be used to carry out the analysis described above: a Quantimet Q520 Image Analyzer (with v. 5.02B software and Grey Store Option), sold by LEICA/Cambridge Instruments Ltd., in conjunction with an Olympus SZH Microscope with a transmitted light base, a plan 1.0.times. objective, and a 2.50.times. eyepiece. The image can be produced with a DAGE MTI CCD72 video camera.

A representative piece of each material to be analyzed is placed on the microscope stage and sharply imaged on the video screen at a microscope zoom setting of 10.times. The open area is determined from field measurements of representative areas. The Quantimet program output reports mean value and standard deviation for each sample.

A suitable starting film for making a three-dimensional apertured film according to the present invention is a thin, continuous, uninterrupted film of thermoplastic polymeric material. This film may be vapor permeable or vapor impermeable; it may be embossed or unembossed; it may be corona-discharge treated on one or both of its major surfaces or it may be free of such corona-discharge treatment; it may be treated with a surface active agent after the film is formed by coating, spraying, or printing the surface active agent onto the film, or the surface active agent may be incorporated as a blend into the thermoplastic polymeric material before the film is formed. The film may comprise any thermoplastic polymeric material including, but not limited to, polyolefins, such as high density polyethylene, linear low density polyethylene, low density polyethylene, polypropylene; copolymers of olefins and vinyl monomers, such as copolymers of ethylene and vinyl acetate or vinyl chloride; polyamides; polyesters; polyvinyl alcohol and copolymers of olefins and acrylate monomers such as copolymers of ethylene and ethyl acrylate and ethylenemethacrylate. Films comprising mixtures of two or more of such polymeric materials may also be used. The machine direction (MD) and cross direction (CD) elongation of the starting film to be apertured should be at least 100% as determined according to ASTM Test No. D-882 as performed on an Instron test apparatus with a jaw speed of 127 cm/minute (50 inches/minute). The thickness of the starting film is preferably uniform and may range from about 12.7 µm (0.5) to about 127 µm (5) mils or about 0.0013 cm (0.0005 inch) to about 0.076 cm (0.005 inch). Coextruded films can be used, as can films that have been modified, e.g., by treatment with a surface active agent. The starting film can be made by any known technique, such as casting, extrusion, or blowing.

A method of aperturing the film involves placing the film onto the surface of a patterned support member. The film is subjected to a high fluid pressure differential as it is on the support member. The pressure differential of the fluid, which may be liquid or gaseous, causes the film to assume the surface pattern of the patterned support member. If the patterned support member has apertures therein, portions of the film overlying the apertures may be ruptured by the fluid pressure differential to create an apertured film. A method of forming an apertured film is described in detail in commonly owned US 5,827,597 to James et al.

Such a three dimensional apertured film is preferably formed by placing a thermoplastic film across the surface of an apertured support member with a pattern corresponding to desired final film shape. A stream of hot air is directed against the film to raise its temperature to cause it to be softened. A vacuum is then applied to the film to cause it to conform to the shape of the surface of the support member. Portions of the film lying over the apertures in the support member are further elongated until rupture to create apertures in the film.

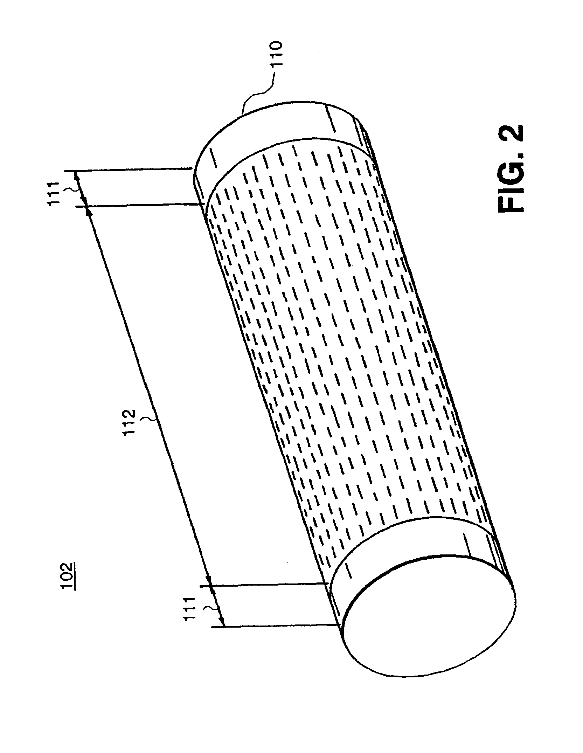

A suitable apertured support member for making these three-dimensional apertured films is a three-dimensional topographical support member made by laser sculpting a workpiece. A schematic illustration of an exemplary workpiece that has been laser sculpted into a three dimensional topographical support member is shown in Figure 2.

The workpiece 102 comprises a thin tubular cylinder 110. The workpiece 102 has non-processed surface areas I I 1 and a laser sculpted center portion 112. A preferred workpiece for producing the support member of this invention is a thin-walled seamless tube of acetal, which has been relieved of all residual internal stresses. The workpiece has a wall thickness of from 1-8 mm, more preferably from 2.5-6.5 mm. Exemplary workpieces for use in forming support members are 30.5 to 182.9 cm (one to six feet) in diameter and have a length ranging from 61.0 cm to 488.0 cm (two to sixteen feet). However, these sizes are a matter of design choice. Other shapes and material compositions may be used for the workpiece, such as acrylics, urethanes, polyesters, high molecular weight polyethylene and other polymers that can be processed by a laser beam.

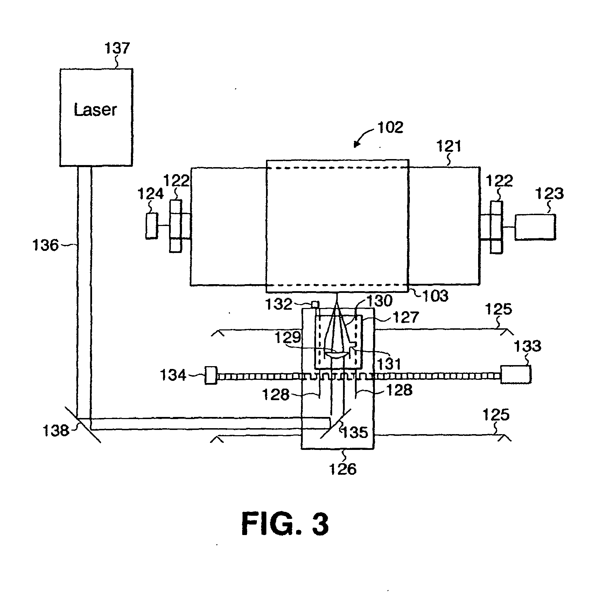

Referring now to Fig. 3, a schematic illustration of an apparatus for laser sculpting the support member is shown. A starting blank tubular workpiece 102 is mounted on an appropriate arbor, or mandrel 121 that fixes it in a cylindrical shape and allows rotation about its longitudinal axis in bearings 122. A rotational drive 123 is provided to rotate mandrel 121 at a controlled rate. Rotational pulse generator 124 is connected to and monitors rotation of mandrel 121 so that its precise radial position is known at all times.

Parallel to and mounted outside the swing of mandrel 121 is one or more guide ways 125 that allow carriage 126 to traverse the entire length of mandrel 121 while maintaining a constant clearance to the top surface 103 of workpiece 102. Carriage drive 133 moves the carriage along guide ways 125, while carriage pulse generator 134 notes the lateral position of the carriage with respect to workpiece 102. Mounted on the carriage is focusing stage 127. Focusing stage 127 is mounted in focus guide ways 128. Focusing stage 127 allows motion orthogonal to that of carriage 126 and provides a means of focusing lens 129 relative to top surface 103. Focus drive 132 is provided to position the focusing stage 127 and provide the focusing of lens 129.

Secured to focusing stage 127 is the lens 129, which is secured in nozzle 130. Nozzle 130 has means 131 for introducing a pressurized gas into nozzle 130 for cooling and maintaining cleanliness of lens 129. A preferred nozzle 130 for this purpose is described in US Patent 5,756,962 to James et al.

Also mounted on the carriage 126 is final bending mirror 135, which directs the laser beam 136 to the focusing lens 129. Remotely located is the laser 137, with optional beam bending mirror 138, to direct the beam to final beam bending mirror 135. While it would be possible to mount the laser 137 directly on carriage 126 and eliminate the beam bending mirrors, space limitations and utility connections to the laser make remote mounting far preferable.

When the laser 137 is powered, the beam 136 emitted is reflected by first beam bending mirror 138, then by final beam bending mirror 135, which directs it to lens 129. The path of laser beam 136 is configured such that, if lens 129 were removed, the beam would pass through the longitudinal center line of mandrel 121. With lens 129 in position, the beam may be focused above, below, at, or near top surface 103.

While this apparatus could be used with a variety of lasers, the preferred laser is a fast flow CO₂ laser, capable of producing a beam rated at up to 2500 watts. However, slow flow CO₂ lasers rated at 50 watts could also be used.

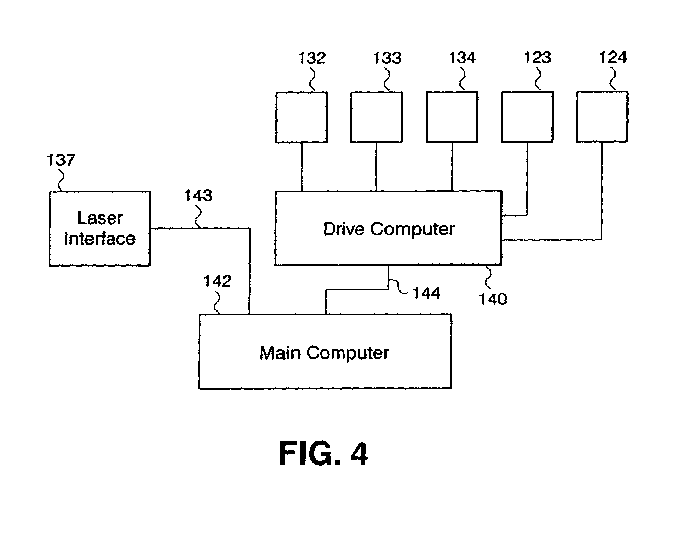

Figure 4 is a schematic illustration of the control system of the laser sculpting apparatus of Figure 3. During operation of the laser sculpting apparatus, control variables for focal position, rotational speed, and traverse speed are sent from a main computer 142 through connection 144 to a drive computer 140. The drive computer 140 controls focus position through focusing stage drive 132. Drive computer 140 controls the rotational speed of the workpiece 102 through rotational drive 123 and rotational pulse generator 124. Drive computer 140 controls the traverse speed of the carriage 126 through carriage drive 133 and carriage pulse generator 134. Drive computer 140 also reports drive status and possible errors to the main computer 142. This system provides positive position control and in effect divides the surface of the workpiece 102 into small areas called pixels, where each pixel consists of a fixed number of pulses of the rotational drive and a fixed number of pulses of the traverse drive. The main computer 142 also controls laser 137 through connection 143.

A laser sculpted three dimensional topographical support member may be made by several methods. One method of producing such a support member is by a combination of laser drilling and laser milling of the surface of a workpiece.

Methods of laser drilling a workpiece include percussion drilling, fire-on-the-fly drilling, and raster scan drilling.

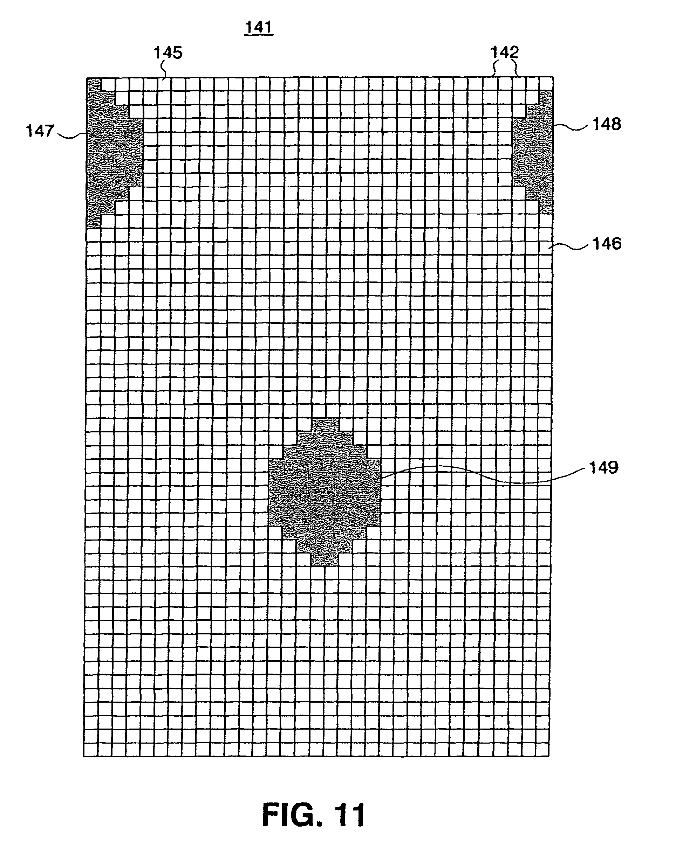

A preferred method is raster scan drilling. In this approach, the pattern is reduced to a rectangular repeat element 141 as depicted in FIG. 11. This repeat element contains all of the information required to produce the desired pattern. When used like a tile and placed both end-to-end and side-by-side, the larger desired pattern is the result.

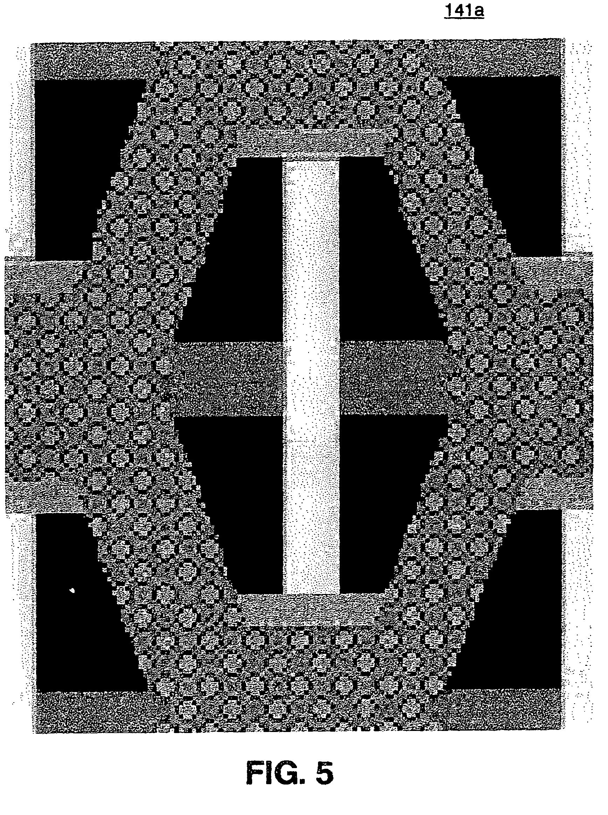

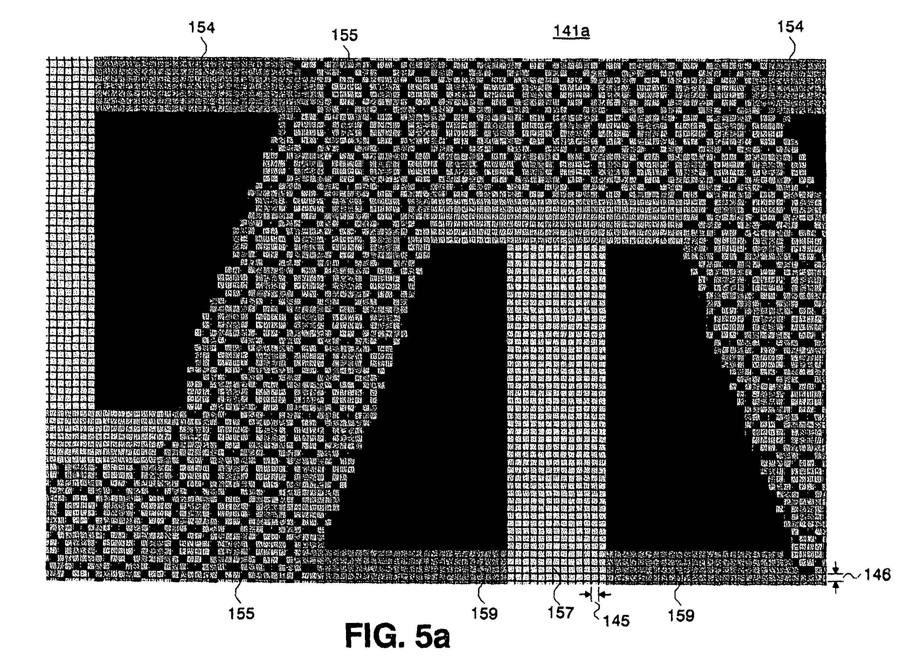

The repeat element 141 is further divided into a grid of smaller rectangular units or "pixels" 142. Though typically square, for some purposes, it may be more convenient to employ pixels of unequal proportions. The pixels themselves are dimensionless and the actual dimensions of the image are set during processing, that is, the width 145 of a pixel and the length 146 of a pixel are only set during the actual drilling operation. During drilling, the length of a pixel is set to a dimension that corresponds to a selected number of pulses from the carriage pulse generator 134. Similarly, the width of a pixel is set to a dimension that corresponds to the number of pulses from the rotational pulse generator 124. Thus, for ease of explanation, the pixels are shown to be square in Figure 5a; however, it is not required that pixels be square, but only that they be rectangular.

Each column of pixels represents one pass of the workpiece past the focal position of the laser. This column is repeated as many times as is required to reach completely around workpiece 102. A white pixel represents an off instruction to the laser and each black pixel represents an on instruction to the laser. This results in a simple binary file of 1's and 0's where a 1, or white, is an instruction for the laser to shut off and a 0, or black, is an instruction for the laser to turn on.

Referring back to Figure 4, the contents of an engraving file are sent in a binary form where 1 is off and 0 is on by the main computer 142 to the laser 137 via connection 143. By varying the time between each instruction, the duration of the instruction is adjusted to conform to the size of the pixel. After each column of the file is completed, that column is again processed, or repeated, until the entire circumference is completed. While the instructions of a column are being carried out, the traverse drive is moved slightly. The speed of traverse is set so that upon completion of a circumferential engraving, the traverse drive has moved the focusing lens the width of a column of pixels and the next column of pixels is processed. This continues until the end of the file is reached and the file is again repeated in the axial dimension until the total desired width is reached.

In this approach, each pass produces a number of narrow cuts in the material, rather than a large hole. Because these cuts are precisely registered to line up side-by-side and overlap somewhat, the cumulative effect is a hole.

A highly preferred method for making the laser sculpted three dimensional topographical support members is through laser modulation. Laser modulation is carried out by gradually varying the laser power on a pixel by pixel basis. In laser modulation, the simple on or off instructions of raster scan drilling are replaced by instructions that adjust on a gradual scale the laser power for each individual pixel of the laser modulation file. In this manner, a three dimensional structure can be imparted to the workpiece in a single pass over the workpiece.

Laser modulation has several advantages over other methods of producing a three dimensional topographical support member. Laser modulation produces a one-piece, seamless, support member without the pattern mismatches caused by the presence of a seam. With laser modulation, the support member is completed in a single operation instead of multiple operations, thus increasing efficiency and decreasing cost. Laser modulation eliminates problems with the registration of patterns, which can be a problem in a multi-step sequential operation. Laser modulation also allows for the creation of topographical features with complex geometries over a substantial distance. By varying the instructions to the laser, the depth and shape of a feature can be precisely controlled and features that continuously vary in cross section can be formed. Also, with laser sculpting the regular positions of the apertures relative to one another can be maintained.

Referring again to Figure 4, during laser modulation the main computer 142 may send instructions to the laser 137 in other than a simple "on" or "off" format. For example, the simple binary file may be replaced with an 8 bit (byte) format, which allows for a variation in power emitted by the laser of 256 possible levels. Utilizing a byte format, the instruction "11111111" instructs the laser to turn off, "00000000" instructs the laser to emit full power, and an instruction such as "10000000" instructs the laser to emit one-half of the total available laser power.

A laser modulation file can be created in many ways. One such method is to construct the file graphically using a gray scale of a 256 color level computer image. In such a gray scale image, black can represent full power and white can represent no power with the varying levels of gray in between representing intermediate power levels. A number of computer graphics programs can be used to visualize or create such a laser-sculpting file. Utilizing such a file, the power emitted by the laser is modulated on a pixel by pixel basis and can therefore directly sculpt a three dimensional topographical support member. While an 8-bit byte format is described here, other levels, such as 4 bit, 16 bit, 24 bit or other formats can be substituted.

A suitable laser for use in a laser modulation system for laser sculpting is a fast flow CO₂ laser with a power output of 2500 watts, although a laser of lower power output could be used. Of primary concern is that the laser must be able to switch power levels as quickly as possible. A preferred switching rate is at least 10 kHz and even more preferred is a rate of 20 kHz. The high power-switching rate is needed to be able to process as many pixels per second as possible.

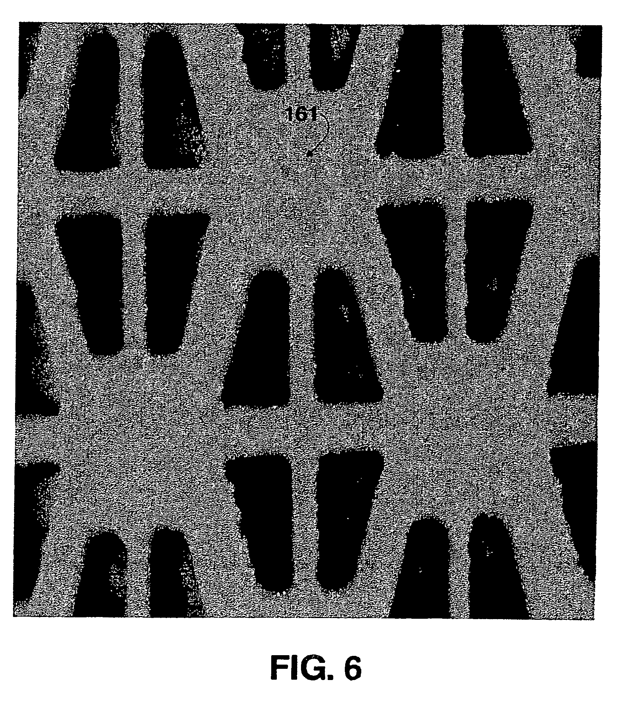

Fig. 5 is a graphical representation of a laser modulation file, including a repeat element 141 a, that may be used to form a support member for forming the apertured film shown in Figs. 1-1a. Fig. 5a is an enlarged portion of the laser modulation file shown in Fig. 5b. In figures 5 and 5a the black areas 154a indicate pixels where the laser is instructed to emit full power, thereby creating a hole in the support member, which corresponds to apertures 16 in the three-dimensional apertured film 10 illustrated in Figs. 1a-1d. The light gray areas 155 in Figs. 5 and 5a indicate pixels where the laser receives instructions to apply a very low level power, thereby leaving the surface of the support member essentially intact. These areas of the support member correspond to the protuberances 11 shown in Fig. 1a. The other areas depicted in Figs. 5 and 5a, which are depicted in various levels of gray, represent corresponding levels of laser power and correspond to various features of the film 10 shown in Figs. 1a-1d. For example, areas 157 and 159 correspond to cross members 14a and 14b of the film 10. Fig. 6 is a photomicropgraph of a portion 161 of a support member after it was engraved using the file shown in Fig. 5. The pattern on the portion of support member shown in Fig. 6 is repeated over the surface of the support member to thereby produce the repeating pattern of the film 10 shown in Figs. 1a-1d.

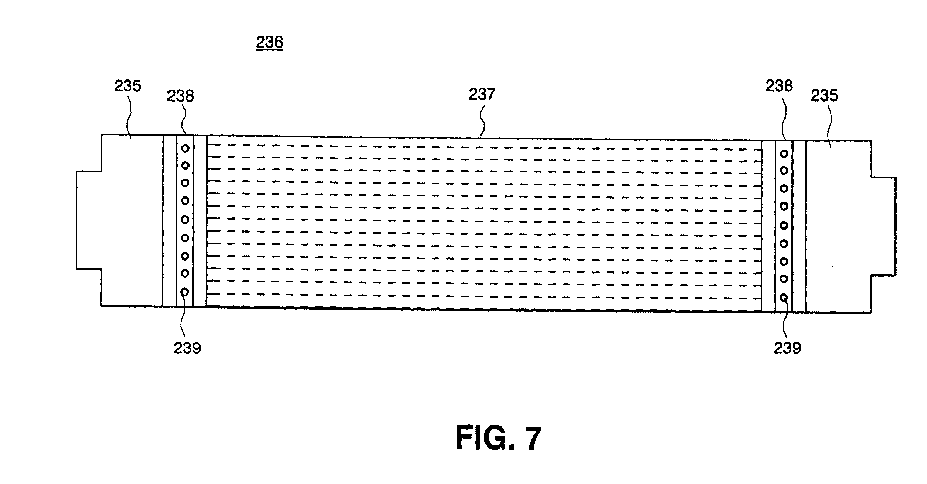

Upon completion of the laser sculpting of the workpiece, it can be assembled into the structure shown in Figure 7 for use as a support member. Two end bells 235 are fitted to the interior of the workpiece 236 with laser sculpted area 237. These end bells can be shrink-fit, press-fit, attached by mechanical means such as straps 238 and screws 239 as shown, or by other mechanical means. The end bells provide a method to keep the workpiece circular, to drive the finished assembly, and to fix the completed structure in the aperturing apparatus.

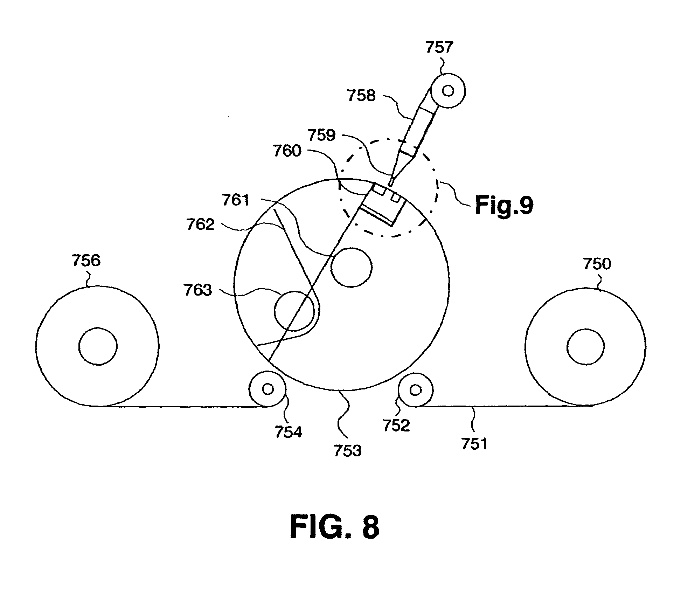

A preferred apparatus for producing such three dimensional apertured films is schematically depicted in Figure 8. As shown here, the support member is a rotatable drum 753. In this particular apparatus, the drum rotates in a counterclockwise direction. Positioned outside drum 753 is a hot air nozzle 759 positioned to provide a curtain of hot air to impinge directly on the film supported by the laser sculpted support member. Means is provided to retract hot air nozzle 759 to avoid excessive heating of the film when it is stopped or moving at slow speed. Blower 757 and heater 758 cooperate to supply hot air to nozzle 759. Positioned inside the drum 753, directly opposite the nozzle 759, is vacuum head 760. Vacuum head 760 is radially adjustable and positioned so as to contact the interior surface of drum 753. A vacuum source 761 is provided to continuously exhaust vacuum head 760.

Cooling zone 762 is provided in the interior of and contacting the inner surface of drum 753. Cooling zone 762 is provided with cooling vacuum source 763. In cooling zone 762, cooling vacuum source 763 draws ambient air through the apertures made in the film to set the pattern created in the aperturing zone. Vacuum source 763 also provides means of holding the film in place in cooling zone 762 in drum 753 and provides means to isolate the film from the effects of tension produced by winding up the film after its aperturing.

Placed on top of laser sculpted support member 753 is a thin, continuous, uninterrupted film 751 of thermoplastic polymeric material.

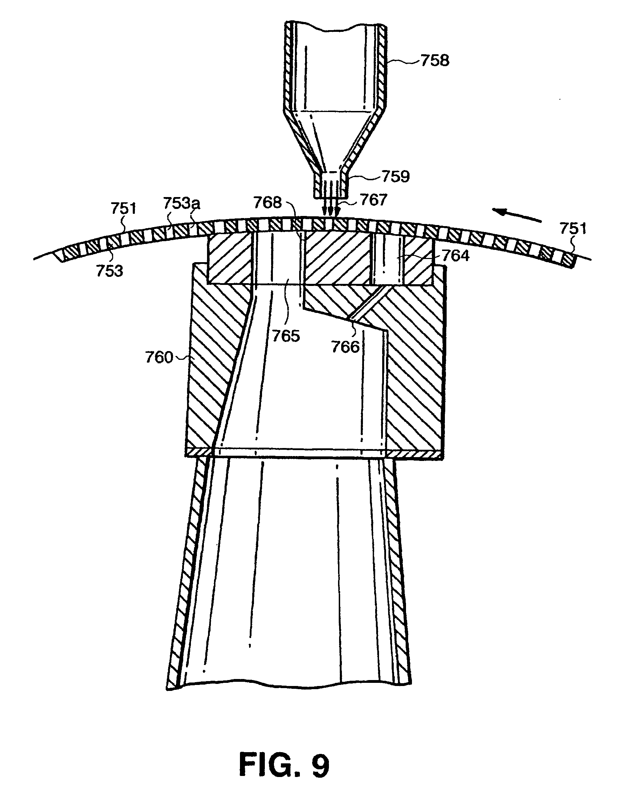

An enlargement of the circled area of Figure 8 is shown in Figure 9. As shown in this embodiment, vacuum head 760 has two vacuum slots 764 and 765 extending across the width of the film. However, for some purposes, it may be preferred to use separate vacuum sources for each vacuum slot. As shown in Figure 23, vacuum slot 764 provides a hold down zone for the starting film as it approaches air knife 758. Vacuum slot 764 is connected to a source of vacuum by a passageway 766. This anchors the incoming film 751 securely to drum 753 and provides isolation from the effects of tension in the incoming film induced by the unwinding of the film. It also flattens film 751 on the outer surface of drum 753. The second vacuum slot 765 defines the vacuum aperturing zone. Immediately between slots 764 and 765 is intermediate support bar 768. Vacuum head 760 is positioned such that the impingement point of hot air curtain 767 is directly above intermediate support bar 768. The hot air is provided at a sufficient temperature, a sufficient angle of incidence to the film, and at a sufficient distance from the film to cause the film to become softened and deformable by a force applied thereto. The geometry of the apparatus ensures that the film 751, when softened by hot air curtain 767, is isolated from tension effects by hold-down slot 764 and cooling zone 762 (Figure 22). Vacuum aperturing zone 765 is immediately adjacent hot air curtain 767, which minimizes the time that the film is hot and prevents excessive heat transfer to support member 753.

Referring to Figures 8 and 9, a thin flexible film 751 is fed from a supply roll 750 over idler roll 752. Roll 752 may be attached to a load cell or other mechanism to control the feed tension of the incoming film 751. The film 751 is then placed in intimate contact with the support member 753. The film and support member then pass to vacuum zone 764. In vacuum zone 764 the differential pressure further forces the film into intimate contact with support member 753. The vacuum pressure then isolates the film from the supply tension. The film and support member combination then passes under hot air curtain 767. The hot air curtain heats the film and support member combination thus softening the film.

The heat-softened film and the support member combination then pass into vacuum zone 765 where the heated film is deformed by the differential pressure and assumes the topography of the support member. The heated film areas that are located over open areas in the support member are further deformed into the open areas of the support member. If the heat and deformation force are sufficient, the film over the open areas of the support member is ruptured to create apertures.

The still-hot apertured film and support member combination then passes to cooling zone 762. In the cooling zone a sufficient quantity of ambient air is pulled through the now-apertured film to cool both the film and the support member.

The cooled film is then removed from the support member around idler roll 754. Idler roll 754 may be attached to a load cell or other mechanism to control winding tension. The apertured film then passes to finish roll 756, where it is wound up.

### Examples

The apertured films according to the present invention improve the transfer of fluids to an underlying absorbent structure while at the same time exhibit improved masking characteristics.

### Construction of Test Assemblies

Test assemblies 1-4 were created to illustrate the improved properties of the apertured film films according to the present invention. Test assemblies #1-#4 were made using a cover layer, transfer layer, absorbent core and barrier layer. Test assemblies #1-4 were constructed from the commercially available sanitary napkin, Stayfree Ultra Thin with Wings, distributed by the Personal Products Company Division of McNeil-PPC, Inc. Skillman, NJ. The cover layer is a thermally bonded polypropylene fabric, the transfer layer is a nonwoven material, the absorbent core is a material containing superabsorbent polymer and the barrier is a pigmented polyethylene film. The cover layer was carefully peeled away from the product exposing the transfer layer.

Test assembly #1 was constructed by first creating an apertured film according to the invention, as show in Figs. 1a-1d and described above (hereinafter referred to as Film #1). Film #1 was constructed such that the upper surfaces of cross members 14a and 14b were recessed relative to the upper surface of film by about 381 µm (15 mils) and the width of the cross members 14a and 14b were about 0.254 µm (.01 mils). Film #1 was measured to have average open area of about 29%. Test assembly #1 was completed by applying Film #1 on top of the exposed transfer layer of the Stayfree Ultra Thin product thus forming an assembly including a cover, transfer layer, core and barrier layer.

Test assembly #2 was constructed by first creating an apertured film (hereinafter referred to as Film #2) that was identical in all respects to Film #1 except for the fact that the cross members 14a and 14b were arranged to be coplanar with the top surface of the film, i.e., the cross members were not recessed relative to the top surface of the film. Film #2 was determined to have an average open area of about 27%. Test assembly #2 was completed by applying the Film #2 on top the exposed transfer layer of the Stayfree Ultra Thin product thus forming an assembly including a cover, transfer layer, core and barrier layer.

Test assembly #3 was constructed by first creating an apertured film (hereinafter referred to as Film #3) that was identical in all respects to Film #1 except for the fact that the cross members 14a and 14b were entirely omitted, i.e., the film included a plurality of hexagonally shaped apertures. Film #3 was measured to have an open area of about 39%. Test assembly #3 was completed by applying Film #3 on top of the exposed transfer layer of the Stayfree Ultra Thin product thus forming an assembly including a cover, transfer layer, core and barrier layer.

Test assembly #4 was constructed by removing an apertured film cover layer (Film #4) from the Sempre Livre Ultra Thin with Wings product manufactured by Johnson & Johnson Ind. E. Com. Ltda. Test assembly was completed by applying Film #4 on the exposed transfer layer of the Stayfree Ultra Thin product thus forming an assembly including a cover, transfer layer, core and barrier layer.

Five samples of each of the test assemblies #1-4 described above were created and tested to determine Fluid Penetration Time (FPT), Rewet (in grams) and Masking Value. The test methods for determining Fluid Penetration Time (FPT), Rewet and Masking Value are discussed in greater detail below. The same five samples were used in each of the tests. That is, a clean sample was not be used for each test but rather the same sample was tested for fluid penetration and then rewet and then masking value.

The test fluid used for the Fluid Penetration test; Rewet test and Masking Value according to the test procedures set forth below may be any synthetic menstrual fluid having the following properties: (1) a viscosity of approximately 0.03 kg m¹s⁻¹ 30 centipoise; and (2) Hunter color values as follows: L = about 17, a = about 7, b = about 1.5. The L Hunter values of the test fluid were measured by placing a quantity of the test fluid in a glass dish to a depth of 0.64 cm (0.25").

### Fluid Penetration Time (FPT)

Fluid Penetration Time is measured by placing a sample to be tested under a Fluid Penetration Test orifice plate. The test plate is rectangular and made from Lexan and is 25.4 cm (10.0 inches) long by 7.6 cm (3.0 inches) wide by 1.27 cm (0.5 inches) thick. A concentric, elliptical orifice is formed through the plate having a major axis of length 3.8 cm and being parallel to the length of the plate and a minor axis of width 1.9 cm and being parallel to the width of the plate.

The orifice plate is centered on the sample to be tested. A graduated 10 cc syringe containing 7 ml of test fluid is held over the orifice plate such that the exit of the syringe is approximately 7.6 cm (3 inches) above the orifice. The syringe is held horizontally, parallel to the surface of the test plate, the fluid is then expelled from the syringe at a rate that allows the fluid to flow in a stream vertical to the test plate into the orifice and a stop watch is started when the fluid first touches the sample to be tested. The stop watch is stopped when surface of the sample first becomes visible within the orifice. The elapsed time on the stop watch is the Fluid Penetration Time. The average Fluid Penetration Time (FPT) is calculated from the results of testing five samples.

### Rewet Potential

The rewet potential is a measure of the ability of a napkin or other article to hold liquid within its structure when the napkin contains a relatively large quantity of liquid and is subjected to external mechanical pressure. The rewet potential is determined and defined by the following procedure.

The apparatus required for the test includes a stop watch with an accuracy to 1 sec and at least 5 minutes duration, a graduated glass cylinder of 10 ml capacity and having an internal diameter of approximately 12 mm, a quantity of test fluid, and a fluid penetration test orifice plate.

The apparatus further includes a weighing machine or balance capable of weighing to an accuracy of +-.0.001 g, a quantity of NuGauze general use sponges (10 cm.times.10 cm) (4 inches.times.4 inches)- 4 ply from Johnson & Johnson Medical Inc. Product Code 3634 (available from Johnson & Johnson Hospital Services, re: order number 7634), a standard weight of 2.22 kg (4.8 pounds) having dimensions 5.1 cm (2 inches) by 10.2 cm (4.0 inches) by approximately 5.4 cm (2.13 inches) which applies a pressure of 4.14 kPa (0.6 psi) over the 5.1 by 10.2 cm (2 inches by 4 inches) surface.

Two sponges are folded with the creased edges placed opposing each other to create a layered structure of approximately 5 cm by 10 cm by 16 plies. A 16 ply sponge for each napkin sample to be tested is then weighed to the nearest 0.001 grams. The preconditioned sanitary napkin or other article is placed on a level surface, without removing the release paper and with the cover layer facing upwards.

After the test fluid is applied within the orifice plate in the FPT test described above, and as soon as the cover layer of the napkin first appears through the top surface of the fluid, the stop watch is started and an interval of 5 minutes is measured. After 5 minutes have elapsed, the orifice plate is removed and the napkin is positioned on a hard level surface with the cover layer facing upwards. One pre-weighed 16 ply layered sponge is placed on and centered over the wetted area and the standard 2.22 kg weight is placed on top of the 16 ply layered sponge. Immediately after placing the sponge and weight on the napkin, the stop watch is started and after a 3 minute interval has elapsed the standard weight and 16 ply layered sponge are quickly removed. The wet weight of the 16 ply layered sponge is measured and recorded to the nearest 0.001 grams. The rewet value is then calculated as the difference in grams between the weight of the wet 16 ply layered sponge and the dry 16 ply layered sponge.

The above measurement is repeated for the five samples and, if necessary, the weight is wiped clean before each run. The average rewet potential is obtained by averaging the value obtained from the five samples.

When conducting the above method, it is important that the tests are performed at a temperature of 21.+-.1.degree. C. and 65.+-.2% relative humidity.

### Masking Value

The following procedure was employed to determine the ability of a facing material to reduce the appearance of product staining after use, i.e., the Masking Value. After each of the assemblies #1-4 were subject to the penetration test and the rewet test, they were immediately imaged, after fluid testing, at 50x using a Scalar USB Microscope model UM02-SUZ-01, utilizing the included light source. The Scalar scope was set at hue saturation and intensity with auto-exposure enabled. Five random areas were imaged from each sample and the images saved as 640 x 480 pixel 24 bit true-color image files in the "bmp" format. Thus a total of 25 images ( 5 images/assembly for each of 5 assemblies) were obtained.

The original "bmp" images were then opened in Image Pro Plus ver 4.0 software, a product of Media Cybermetics, LP. The images were then converted, in Image Pro Plus, from their original 24 bit true-color format into an 8-bit gray scale image. Image Pro Plus's histogram function was then applied to the images and a histogram of the images gray values was then constructed. This provides a count of the number of pixels at a particular gray value which gray value ranges from "0" black to "255" white. The data from the histogram was then transferred into a Microsoft Excel 2000 worksheet, utilizing DDE (Windows dynamic data exchange).

The DDE to Excel 2000 then produces a worksheet that contains 25 columns each containing 256 rows. Each of the columns in the worksheet contains the histogram values for a single image. Each column consists of 256 values, which is a count of the number of pixels in the image, which have a corresponding value from 0 to 255. Each of the rows was then averaged to create an average histogram for that particular material.

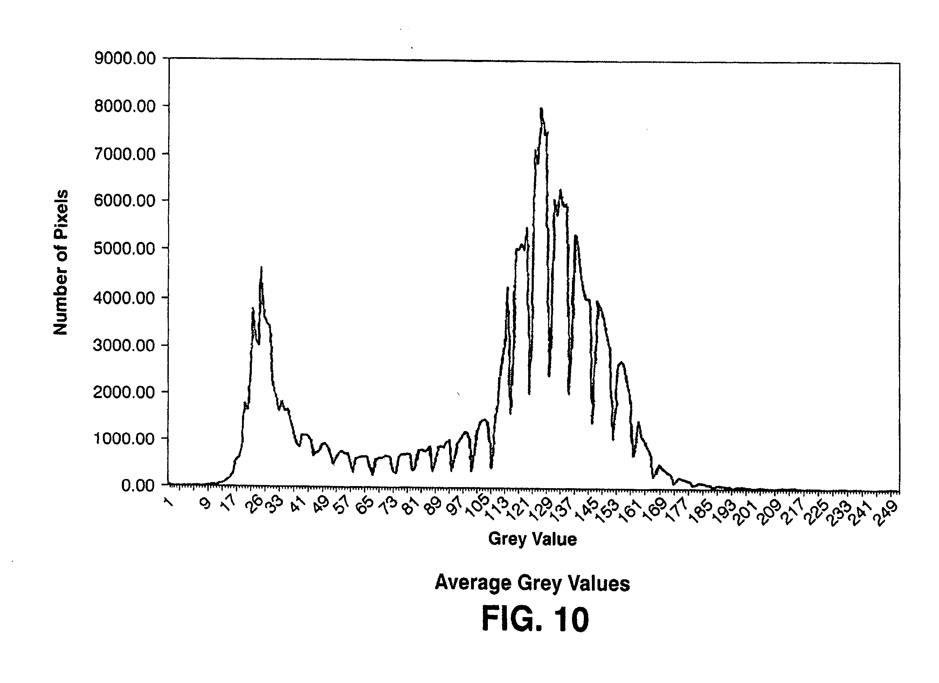

A typical average histogram shows a bi-modal distribution of the gray area, representing the stained area of the test assembly, and the white area, representing the unstained area of the test assembly. Examination of the average histograms demonstrated a plateau between the gray region and the white region and that all of the stained area was defined by a gray value of 90 or less. Thus, the stain area of a material can be determined by the sum of gray values between 0 and 90, with lower values representing lower gray areas and thus better masking. The summation of the gray values of 90 or less is the "Masking Value". Fig. 10 is a typcal average histogram representing stain intensity for an absorbent article having a apertured film according to the present invention as the cover layer thereof.

**Table 1 set forth below provides the average Fluid Penetration Time, average Rewet (in grams) and Masking Value for test assemblies #1-#3.**

| **Test Assembly** | **Average Fluid Penetration Time (in seconds)** | **Average Rewet (in grams)** | **Masking Value** |
|---|---|---|---|
| #1 | 35.08 | .030 | 84,469.82 |
| #2 | 45.52 | .040 | 78,587.00 |
| #3 | 21.55 | .052 | 114,930.20 |
| #4 | 46.50 | .024 | 111,959.93 |

As set forth in the table above, the test assembly #1 constructed using the apertured film according to the present invention provides a unique combination of fluid handling capabilities and masking characteristics.

Although specific embodiments of the invention have been described above, it is intended that the present application cover the modifications and variations of the invention provided that they come with the scope of the appended claims.

## Claims

1. A three dimensional apertured film (10) comprising:
a first planar surface (18) in a first imaginary plane (23);
a second planar surface (21) in a second imaginary plane (25) located below said first imaginary plane;
a first plurality of apertures (16);
at least one member spanning (14) each one of said first plurality of apertures (16) to thereby define a plurality of smaller apertures, each of said plurality of smaller apertures in communication with a respective one of said first plurality of apertures, wherein said member (14) spanning each one of said apertures (16) has a top surface (44) located in a third imaginary plane (27), said third imaginary plane being located below said first imaginary plane (23) and said second imaginary plane, and a bottom surface located in a fourth imaginary plane (29) located below the first, second and third imaginary planes.

2. The three dimensional apertured film according to claim 1, wherein said first plurality of apertures are defined from said first imaginary plane to said third imaginary plane.

3. The three dimensional apertured film (10) according to claim 1, wherein said top surface of said member (44) spanning each one of said plurality of apertures (16) is substantially parallel to said first imaginary plane (23) and said second imaginary plane (25).

4. The three dimensional apertured film (10) according to claim 1, wherein said third imaginary (27) plane is located below said first (23) and second (25) imaginary planes.

5. The three dimensional apertured film (10) according to claim 1, wherein said at least one member (14) spanning each one of said plurality of apertures (16) comprises:
a first cross member (14a) spanning each one of said plurality apertures; and
a second cross member (14b) spanning each one of said plurality of apertures.

6. The three dimensional apertured film (10) according to claim 5, wherein said first cross member (14a) intersects said second cross (14b) member.

7. The three dimensional apertured film (10) according to claim 6, wherein said first cross member (14a) and second cross member (14b) are orthongally arranged with respect to one another.

8. The three dimensional apertured film (10) according to claim 1, wherein said apertured film has a plurality of bumps arranged on said first planar surface (18).

9. The three dimensional apertured film (10) according to claim 1, wherein each of said members (14) spanning each one of said first plurality of apertures has a width in the range of about .008 mils to about .024 mils.

10. The three dimensional apertured film (10) according to claim 5, wherein said first (14a) and second (14b) cross members each have a width in the range of about .008 mils to about .024 mils.

11. The three dimensional apertured film (10) according to claim 1, wherein said film has an open area in the range of about 20% to about 30%.

12. The three dimensional apertured film (10) of claim 1 wherein it comprises
a plurality of interconnected frame portions (12), each of said frame portions having at least first (22) and second (24) interior walls arranged in opposed spaced relationship to one another;
a plurality of cross members (14), each one of said cross members extending from one of said interior walls of one of said frame portions to said opposed second interior wall of one of said frame portions, each of said cross members having a top surface (42, 44) located in a imaginary plane (27) located below said first imaginary plane (23);
a plurality of apertures (16) extending from at least said first planar surface (18) to said second planar surface (21), each of said apertures being bound by at least one of said frame portions (12) and at least one of said cross members (14).

13. The three dimensional apertured film (10) according to claim 12, wherein said top surface (44) of each one of said cross members (14) is substantially parallel to said first imaginary plane (23) and said second imaginary plane (25).

14. The three dimensional apertured film (10) according to claim 13, wherein said top surface (44) of each one of said cross members (14) is located in a third imaginary plane (27) below said first imaginary plane (23) and said second imaginary plane (25).

15. The three dimensional apertured film (10) according to claim 12, wherein each of said frame portions (12) includes a opposed spaced end regions (12a, 12b) and opposed spaced side walls (12c, 12d).

16. The three dimensional apertured film (10) according to claim 15, wherein said plurality of cross members (14) comprises:
a first plurality of cross members (14a), each one of said first plurality of cross members extending from one of said end regions (12a, 12b) of said frame to an opposed end region (12a, 12b) of said frame (12); and
a second plurality of cross members (14b), each one of said second plurality of cross members extending from one of said side walls (12c, 12d) of said frame to an opposed side wall (12c, 12d) of said frame (12).

17. The three dimensional apertured film (10) according to claim 16, wherein each of said first plurality of cross members (14a) intersects with one of said second plurality of cross members (14b).

18. The three dimensional apertured film (10) according to claim 17, wherein each of said first plurality of cross members (14a) is orthoganally arranged with respect to one of said second plurality of cross members (14b).

19. The three dimensional apertured film (10) according to claim 12, wherein each of said frame portions (12) is substantially a hexagon in shape.

20. The three dimensional film (10) according to claim 12, further comprising:
a plurality of bumps extending upwardly from said first planar surface (18) of said film.

21. The three dimensional apertured film (10) according to claim 12, wherein said film has an open area in the range of about 20% to about 30%.

22. The three dimensional apertured film (10) of claim 1 wherein it comprises
the plurality of apertures (16) extending at least from said first planar surface (18) to said second planar surface (21);

23. The three dimensional apertured film (10) according to claim 22, wherein said top surface (44) of said member (14) spanning each one of said plurality of apertures (16) is substantially parallel to said first imaginary plane (23) and said second imaginary plane (25).

24. The three dimensional apertured film (10) according to claim 22, wherein said third imaginary plane (27) is located below said first (23) and second (25) imaginary planes.

25. The three dimensional apertured film (10) according to claim 22, wherein said at least one member (14) spanning each one of said plurality of apertures (16) comprises:
a first cross member (14a) spanning each one of said plurality apertures (16); and
a second cross member (14b) spanning each one of said plurality of apertures (16).

26. The three dimensional apertured film (10) according to claim 25, wherein said first cross member (14a) intersects said second cross member (14b).

27. The three dimensional apertured film (10) according to claim 26, wherein said first cross member (14a) and second cross member (14b) are orthogonally arranged with respect to one another.

28. The three dimensional apertured film (10) according to claim 22, wherein said apertured film (10) has a plurality of bumps arranged on said first planar surface (18).

29. The three dimensional apertured film (10) according to claim 22, wherein each of said members (14) spanning each one of said first plurality of apertures (16) has a width in the range of about .008 mils to about .024 mils.

30. The three dimensional apertured film (10) according to claim 25, wherein said first (14a) and second (14b) cross members each have a width in the range of about .008 mils to about .024 mils.

31. The three dimensional apertured film (10) according to claim 22, wherein said film has an open area in the range of about 20% to about 30%.

32. The three dimensional apertured film (10) according to claim 1, wherein said apertured film is a cover layer in an absorbent article.

33. The three dimensional apertured film (10) according to claim 12, wherein said apertured film is a cover layer in an absorbent article.

34. The three dimensional apertured film (10) according to claim 22, wherein said apertured film is a cover layer in an absorbent article.

## Patentansprüche

1. Dreidimensionaler perforierter Film (10), umfassend:
eine erste planare Oberfläche (18) in einer ersten imaginären Ebene (23);
eine zweite planare Oberfläche (21) in einer zweiten imaginären Ebene (25), die unterhalb der ersten imaginären Ebene angeordnet ist;
eine erste Vielzahl von Öffnungen (16);
wenigstens ein Element (14), das sich über jede der Vielzahl von Öffnungen (16) erstreckt, um **dadurch** eine Vielzahl von kleineren Öffnungen zu definieren, wobei jede der Vielzahl von kleineren Öffnungen mit einer entsprechenden der ersten Vielzahl von Öffnungen in Kontakt steht, wobei das Element (14), das sich über jede der Öffnungen (16) erstreckt, eine Oberseite (44) aufweist, die in einer dritten imaginären Ebene (27) angeordnet ist, wobei die dritte imaginäre Ebene unterhalb der ersten imaginären Ebene (23) und der zweiten imaginären Ebene angeordnet ist; und
eine Unterseite, die in einer vierten imaginären Ebene (29) angeordnet ist, die unterhalb der ersten, zweiten und dritten imaginären Ebenen angeordnet ist.

2. Dreidimensionaler perforierter Film nach Anspruch 1, **dadurch gekennzeichnet, dass** die erste Vielzahl von Öffnungen von der ersten imaginären Ebene zu der dritten imaginären Ebene definiert sind.

3. Dreidimensionaler perforierter Film (10) nach Anspruch 1, **dadurch gekennzeichnet, dass** die Oberseite des Elements (44), das sich über jede der Vielzahl von Öffnungen (16) erstreckt, im wesentlichen parallel zu der ersten imaginären Ebene (23) und der zweiten imaginären Ebene (25) ist.

4. Dreidimensionaler perforierter Film (10) nach Anspruch 1, **dadurch gekennzeichnet, dass** die dritte imaginäre Ebene unterhalb der ersten (23) und zweiten (25) imaginären Ebenen angeordnet ist.

5. Dreidimensionaler perforierter Film (10) nach Anspruch 1, **dadurch gekennzeichnet, dass** das wenigstens eine Element (14), das sich über jede der Vielzahl von Öffnungen (16) erstreckt, umfasst:
ein erstes Kreuzelement (14a), das sich über jede der Vielzahl von Öffnungen erstreckt; und
ein zweites Kreuzelement (14b), das sich über jede der Vielzahl von Öffnungen erstreckt.

6. Dreidimensionaler perforierter Film (10) nach Anspruch 5, **dadurch gekennzeichnet, dass** das erste Kreuzelement (14a) das zweite Kreuzelement (14b) schneidet.

7. Dreidimensionaler perforierter Film (10) nach Anspruch 6, **dadurch gekennzeichnet, dass** das erste Kreuzelement (14a) und das zweite Kreuzelement (14b) zueinander orthogonal angeordnet sind.

8. Dreidimensionaler perforierter Film (10) nach Anspruch 1, **dadurch gekennzeichnet, dass** der perforierte Film eine Vielzahl von Erhebungen aufweist, die auf der ersten ebenen Oberfläche (18) angeordnet sind.

9. Dreidimensionaler perforierter Film (10) nach Anspruch 1, **dadurch gekennzeichnet, dass** jedes der Elemente (14), die sich über jede der Vielzahl von Öffnungen erstrecken, eine Breite im Bereich von etwa 0,008 mil (0,2032 µm) bis etwa 0,024 mil (0,6096 µm) aufweist.

10. Dreidimensionaler perforierter Film (10) nach Anspruch 5, **dadurch gekennzeichnet, dass** die ersten (14a) und zweiten (14b) Kreuzelemente jeweils eine Breite im Bereich von etwa 0,008 mil (0,2032 µm) bis etwa 0,024 mil (0,6096 µm) aufweisen.

11. Dreidimensionaler perforierter Film (10) nach Anspruch 1, **dadurch gekennzeichnet, dass** der Film eine Freifläche im Bereich von etwa 20 % bis etwa 30 % aufweist.

12. Dreidimensionaler perforierter Film (10) nach Anspruch 1, **dadurch gekennzeichnet, dass** dieser ferner umfasst:
eine Vielzahl miteinander verbundener Rahmenabschnitte (12), wobei jeder der Rahmenabschnitte wenigstens erste (22) und zweite (24) innere Wände aufweist, die in einander gegenüberliegender beabstandeter Beziehung zueinander angeordnet sind;
eine Vielzahl von Kreuzelementen (14), wobei sich jedes der Kreuzelemente von einer der inneren Wände eines der Rahmenabschnitte zu der gegenüberliegenden zweiten inneren Wand eines der Rahmenabschnitte erstreckt, wobei jedes der Kreuzelemente eine Oberseite (42, 44) aufweist, die in einer imaginären Ebene (27) angeordnet ist, die unterhalb der ersten imaginären Ebene (23) angeordnet ist;
eine Vielzahl von Öffnungen (16), die sich von wenigstens der ersten ebenen Oberfläche (18) zu der zweiten ebenen Oberfläche (21) erstrecken, wobei jede der Öffnungen von wenigstens einem der Rahmenabschnitte (12) und wenigstens einem der Kreuzelemente (14) begrenzt ist.

13. Dreidimensionaler perforierter Film (10) nach Anspruch 12, **dadurch gekennzeichnet, dass** die Oberseite (44) jedes Kreuzelements (14) im wesentlichen parallel zu der ersten imaginären Ebene (23) und der zweiten imaginären Ebene (25) ist.

14. Dreidimensionaler perforierter Film (10) nach Anspruch 13, **dadurch gekennzeichnet, dass** die Oberseite (44) jedes Kreuzelements (14) in einer dritten imaginären Ebene (27) unterhalb der ersten imaginären Ebene (23) und der zweiten imaginären Ebene (25) angeordnet ist.

15. Dreidimensionaler perforierter Film (10) nach Anspruch 12, **dadurch gekennzeichnet, dass** jeder der Rahmenabschnitte (12) einander gegenüberliegende beabstandete Endregionen (12a, 12b) und einander gegenüberliegende beabstandete Seitenwände (12c, 12d) aufweist.

16. Dreidimensionaler perforierter Film (10) nach Anspruch 15, **dadurch gekennzeichnet, dass** die Vielzahl der Kreuzelemente (14) umfasst:
eine erste Vielzahl von Kreuzelementen (14a), wobei sich jedes der ersten Vielzahl von Kreuzelementen von einer der Endregionen (12a, 12b) des Rahmens zu einer gegenüberliegenden Endregion (12a, 12b) des Rahmens (12) erstreckt; und
eine zweite Vielzahl von Kreuzelementen (14b), wobei sich jedes der zweiten Vielzahl von Kreuzelementen von einer der Seitenwände (12c, 12d) des Rahmens zu einer gegenüberliegenden Seitenwand (12c, 12d) des Rahmens (12) erstreckt.

17. Dreidimensionaler perforierter Film (10) nach Anspruch 16, **dadurch gekennzeichnet, dass** jedes der ersten Vielzahl von Kreuzelementen (14a) sich mit einem der zweiten Vielzahl von Kreuzelementen (14b) schneidet.

18. Dreidimensionaler perforierter Film (10) nach Anspruch 17, **dadurch gekennzeichnet, dass** jedes der ersten Vielzahl von Kreuzelementen (14a) orthogonal bezüglich einem der zweiten Vielzahl von Kreuzelementen (14b) angeordnet ist.

19. Dreidimensionaler perforierter Film (10) nach 12, **dadurch gekennzeichnet, dass** jeder der Rahmenabschnitte (12) im wesentlichen die Form eines Hexagons aufweist.

20. Dreidimensionaler perforierter Film (10) nach Anspruch 12, ferner umfassend:
eine Vielzahl von Erhebungen, die sich von der ersten planaren Oberfläche (18) des Films nach oben erstrecken.

21. Dreidimensionaler perforierter Film (10) nach Anspruch 12, **dadurch gekennzeichnet, dass** der Film eine Freifläche im Bereich von etwa 20 % bis etwa 30 % aufweist.

22. Dreidimensionaler perforierter Film (10) nach Anspruch 1, **dadurch gekennzeichnet, dass** dieser umfasst:
eine Vielzahl von Öffnungen (16), die sich wenigstens von der ersten planaren Oberfläche (18) zu der zweiten planaren Oberfläche (21) erstrecken.

23. Dreidimensionaler perforierter Film (10) nach Anspruch 22, **dadurch gekennzeichnet, dass** die Oberfläche (44) der Elemente (14), die sich über jede der Vielzahl von Öffnungen (16) erstrecken, im wesentlichen parallel zu der ersten imaginären Ebene (23) und der zweiten imaginären Ebene (25) ist.

24. Dreidimensionaler perforierter Film (10) nach Anspruch 22, **dadurch gekennzeichnet, dass** die dritte imaginäre Ebene (27) unterhalb der ersten (23) und zweiten (25) imaginären Ebenen angeordnet ist.

25. Dreidimensionaler perforierter Film (10) nach Anspruch 22, **dadurch gekennzeichnet, dass** das wenigstens eine Element (14), das sich über jede der Vielzahl von Öffnungen (16) erstreckt, umfasst:
ein erstes Kreuzelement (14a), das sich über jede der Vielzahl von Öffnungen (16) erstreckt; und
ein zweites Kreuzelement (14b), das sich über jede der Vielzahl von Öffnungen (16) erstreckt.

26. Dreidimensionaler perforierter Film (10) nach Anspruch 25, **dadurch gekennzeichnet, dass** das erste Kreuzelement (14a) das zweite Kreuzelement (14b) schneidet.

27. Dreidimensionaler perforierter Film (10) nach Anspruch 26, **dadurch gekennzeichnet, dass** das erste Kreuzelement (14a) und das zweite Kreuzelement (14b) zueinander orthogonal angeordnet sind.

28. Dreidimensionaler perforierter Film (10) nach Anspruch 22, **dadurch gekennzeichnet, dass** der perforierte Film (10) eine Vielzahl von Erhebungen aufweist, die auf der ersten planaren Oberfläche (18) angeordnet sind.

29. Dreidimensionaler perforierter Film (10) nach Anspruch 22, **dadurch gekennzeichnet, dass** jedes der Elemente (14), die sich über jede der ersten Vielzahl von Öffnungen (16) erstrecken, eine Breite im Bereich von etwa 0,008 mil (0,2032 µm) bis etwa 0,024 mil (0,6096 µm) aufweisen.

30. Dreidimensionaler perforierter Film (10) nach Anspruch 25, **dadurch gekennzeichnet, dass** die ersten (14a) und zweiten (14b) Kreuzelemente je eine Breite im Bereich von etwa 0,008 mil (0,2032 µm) bis etwa 0,024 mil (0,6096 µm) aufweisen.

31. Dreidimensionaler perforierter Film (10) nach Anspruch 22, **dadurch gekennzeichnet, dass** der Film eine Freifläche im Bereich von etwa 20 % bis etwa 30 % aufweist.

32. Dreidimensionaler perforierter Film (10) nach Anspruch 1, **dadurch gekennzeichnet, dass** der perforierte Film eine Deckschicht in einem absorbierenden Erzeugnis ist.

33. Dreidimensionaler perforierter Film (10) nach Anspruch 12, **dadurch gekennzeichnet, dass** der perforierte Film eine Deckschicht in einem absorbierenden Erzeugnis ist.

34. Dreidimensionaler perforierter Film (10) nach Anspruch 22, **dadurch gekennzeichnet, dass** der perforierte Film eine Deckschicht in einem absorbierenden Erzeugnis ist.

## Revendications

1. Film ajouré tridimensionnel (10) comprenant :
■ une première surface plane (18) située dans un premier plan imaginaire (23) ;
■ une deuxième surface plane (21) située dans un deuxième plan imaginaire (25) situé en dessous dudit premier plan imaginaire ;
■ une première pluralité d'ouvertures (16) ;
■ au moins un élément (14) qui chevauche chacune de ladite première pluralité d'ouvertures (16) de manière à définir de ce fait une pluralité d'ouvertures plus petites, chacune de ladite pluralité d'ouvertures plus petites étant en communication avec l'une respective de ladite première pluralité d'ouvertures, dans lequel ledit élément (14) qui chevauche chacune desdites ouvertures (16) présente une surface supérieure (44) située dans un troisième plan imaginaire (27), ledit troisième plan imaginaire étant situé en dessous dudit premier plan imaginaire (23) et dudit deuxième plan imaginaire (25), et une surface inférieure située dans un quatrième plan imaginaire (29) situé en dessous dudit premier, dudit deuxième et dudit troisième plans imaginaires.

2. Film ajouré tridimensionnel selon la revendication 1, dans lequel ladite première pluralité d'ouvertures sont définies à partir dudit premier plan imaginaire vers ledit troisième plan imaginaire.

3. Film ajouré tridimensionnel (10) selon la revendication 1, dans lequel ladite surface supérieure (44) dudit élément (14) qui chevauche chacune de ladite pluralité d'ouvertures (16) est sensiblement parallèle audit premier plan imaginaire (23) et audit deuxième plan imaginaire (25).

4. Film ajouré tridimensionnel (10) selon la revendication 1, dans lequel ledit troisième plan imaginaire (27) se situe en dessous dudit premier (23) et dudit deuxième (25) plans imaginaires.

5. Film ajouré tridimensionnel (10) selon la revendication 1, dans lequel ledit au moins un élément (14) qui chevauche chacune de ladite pluralité d'ouvertures (16) comprend :
■ un premier élément transversal (14a) qui chevauche chacune de ladite pluralité d'ouvertures ; et
■ un deuxième élément transversal (14b) qui chevauche chacune de ladite pluralité d'ouvertures.

6. Film ajouré tridimensionnel (10) selon la revendication 5, dans lequel ledit premier élément transversal (14a) croise ledit deuxième élément transversal (14b).

7. Film ajouré tridimensionnel (10) selon la revendication 6, dans lequel ledit premier élément transversal (14a) et ledit deuxième élément transversal (14b), sont disposés de manière orthogonale l'un par rapport à l'autre.

8. Film ajouré tridimensionnel (10) selon la revendication 1, dans lequel ledit film ajouré présente une pluralité de bosses disposées sur ladite première surface plane (18).

9. Film ajouré tridimensionnel (10) selon la revendication 1, dans lequel chacun desdits éléments (14) qui chevauche chacune de ladite première pluralité d'ouvertures, présente une largeur comprise entre 0,008 mil environ et 0,024 mil environ.

10. Film ajouré tridimensionnel (10) selon la revendication 5, dans lequel lesdits premier (14a) et deuxième (14b) éléments transversaux présentent chacun une largeur qui se situe dans une plage comprise entre 0,008 mil environ et 0,024 mil environ.

11. Film ajouré tridimensionnel (10) selon la revendication 1, dans lequel ledit film présente une surface ouverte qui se situe dans une plage comprise entre 20 % environ et 30 % environ.

12. Film ajouré tridimensionnel (10) selon la revendication 1, comprenant :
■ une pluralité de parties de cadre (12) interconnectées, chacune desdites parties de cadre ayant au moins des première (22) et deuxième (24) parois intérieures disposées dans une relation espacée opposée l'une par rapport à l'autre ;
■ une pluralité d'éléments transversaux (14), chacun desdits éléments transversaux s'étendant à partir de l'une desdites parois intérieures de l'une desdites parties de cadre, vers ladite deuxième paroi intérieure opposée de l'une desdites parties de cadre, chacun desdits éléments transversaux présentant une surface supérieure (42, 44) située dans un plan imaginaire (27) situé en dessous dudit premier plan imaginaire (23) ;
■ une pluralité d'ouvertures (16) qui s'étendent à partir d'au moins ladite première surface plane (18) vers ladite deuxième surface plane (21), chacune desdites ouvertures étant entourée par au moins l'une desdites parties de cadre (12) et au moins l'un desdits éléments transversaux (14).

13. Film ajouré tridimensionnel (10) selon la revendication 12, dans lequel ladite surface supérieure (44) de chacun desdits éléments transversaux (14), est sensiblement parallèle audit premier plan imaginaire (23) et audit deuxième plan imaginaire (25).

14. Film ajouré tridimensionnel (10) selon la revendication 13, dans lequel ladite surface supérieure (44) de chacun desdits éléments transversaux (14), se situe dans un troisième plan imaginaire (27) situé en dessous dudit premier plan imaginaire (23) et dudit deuxième plan imaginaire (25).

15. Film ajouré tridimensionnel (10) selon la revendication 12, dans lequel chacune desdites parties de cadre (12) comprend des régions d'extrémité espacées opposées (12a, 12b) et des parois latérales espacées opposées (12c, 12d).

16. Film ajouré tridimensionnel (10) selon la revendication 15, dans lequel ladite pluralité d'éléments transversaux (14) comprend :
■ une première pluralité d'éléments transversaux (14a), chacun de ladite première pluralité d'éléments transversaux s'étendant à partir de l'une desdites régions d'extrémité (12a, 12b) dudit cadre, vers une région d'extrémité opposée (12a, 12b) dudit cadre (12) ; et
■ une deuxième pluralité d'éléments transversaux (14b), chacun de ladite deuxième pluralité d'éléments transversaux s'étendant à partir de l'une desdites parois latérales (12c, 12d) dudit cadre, vers une paroi latérale opposée (12c, 12d) dudit cadre (12).

17. Film ajouré tridimensionnel (10) selon la revendication 16, dans lequel chacun de ladite première pluralité d'éléments transversaux (14a), croise l'un de ladite deuxième pluralité d'éléments transversaux (14b).

18. Film ajouré tridimensionnel (10) selon la revendication 17, dans lequel chacun de ladite première pluralité d'éléments transversaux (14a), est disposé de manière orthogonale par rapport à l'un de ladite deuxième pluralité d'éléments transversaux (14b).

19. Film ajouré tridimensionnel (10) selon la revendication 12, dans lequel chacune desdites parties de cadre (12) présente une forme sensiblement hexagonale.

20. Film tridimensionnel (10) selon la revendication 12, comprenant en outre une pluralité de bosses qui s'étendent vers le haut à partir de ladite première surface plane (18) dudit film.

21. Film ajouré tridimensionnel (10) selon la revendication 12, dans lequel ledit film présente une surface ouverte qui se situe dans une plage comprise entre 20 % environ et 30 % environ.

22. Film ajouré tridimensionnel (10) selon la revendication 1, comprenant une pluralité d'ouvertures (16) qui s'étendent au moins à partir de ladite première surface plane (18) vers ladite deuxième surface plane (21).

23. Film ajouré tridimensionnel (10) selon la revendication 22, dans lequel ladite surface supérieure (44) dudit élément (14) qui chevauche chacune de ladite pluralité d'ouvertures (16) est sensiblement parallèle audit premier plan imaginaire (23)et audit deuxième plan imaginaire (25).

24. Film ajouré tridimensionnel (10) selon la revendication 22, dans lequel ledit troisième plan imaginaire (27) se situe en dessous dudit premier (23) et dudit deuxième (25) plans imaginaires.

25. Film ajouré tridimensionnel (10) selon la revendication 22, dans lequel ledit au moins un élément (14) qui chevauche chacune de ladite pluralité d'ouvertures (16) comprend :
■ un premier élément transversal (14a) qui chevauche chacune de ladite pluralité d'ouvertures (16) ; et
■ un deuxième élément transversal (14b) qui chevauche chacune de ladite pluralité d'ouvertures (16).

26. Film ajouré tridimensionnel (10) selon la revendication 25, dans lequel ledit premier élément transversal (14a) croise ledit deuxième élément transversal (14b).

27. Film ajouré tridimensionnel (10) selon la revendication 26, dans lequel ledit premier élément transversal (14a) et ledit deuxième élément transversal (14b) sont disposés de manière orthogonale l'un par rapport à l'autre.

28. Film ajouré tridimensionnel (10) selon la revendication 22, dans lequel ledit film ajouré (10) présente une pluralité de bosses disposées sur ladite première surface plane (18).

29. Film ajouré tridimensionnel (10) selon la revendication 22, dans lequel chacun desdits éléments (14) qui chevauche chacune de ladite première pluralité d'ouvertures (16) présente une largeur comprise entre 0,008 mil environ et 0,024 mil environ.

30. Film ajouré tridimensionnel (10) selon la revendication 25, dans lequel lesdits premier (14a) et deuxième (14b) éléments transversaux présentent chacun une largeur qui se situe dans une plage comprise entre 0,008 mil environ et 0,024 mil environ.

31. Film ajouré tridimensionnel (10) selon la revendication 22, dans lequel ledit film présente une surface ouverte qui se situe dans une plage comprise entre 20 % environ et 30 % environ.

32. Film ajouré tridimensionnel (10) selon la revendication 1, dans lequel ledit film ajouré est une couche de recouvrement dans un article absorbant.

33. Film ajouré tridimensionnel (10) selon la revendication 12, dans lequel ledit film ajouré est une couche de recouvrement dans un article absorbant.

34. Film ajouré tridimensionnel (10) selon la revendication 22, dans lequel ledit film ajouré est une couche de recouvrement dans un article absorbant.
